# EUROPEAN PATENT SPECIFICATION

(11) **EP 2 303 139 B1**
(45) Date of publication and mention of the grant of the patent: **18.09.2019**
(21) Application number: 09795126.3
(22) Date of filing: 08.07.2009
(51) Int. Cl.: A61B 34/30, A61B 17/00

(54) **ROBOTIC SURGICAL SYSTEM COMPRISING A SURGICAL ATTACHMENT**
ROBOTERCHIRURGIESYSTEM MIT EINEM CHIRURGISCHEN AUFSATZ
SYSTÈME CHIRURGICAL ROBOTISÉ AVEC FIXATION CHIRURGICALE

(30) Priority: 08.07.2008 US 79045 P; 09.07.2008 US 79416 P
(43) Date of publication of application: 06.04.2011
(73) Proprietor: Covidien LP, Mansfield, MA 02048 (US)
(72) Inventor: WHITMAN, Michael P., New Hope, PA 18938 (US); MALINOUSKAS, Donald, Monroe, CT 06468 (US)
(74) Representative: Maschio, Antonio
(86) International application number: PCT/US2009/049947
(87) International publication number: WO 2010/006057

(56) References cited:
- US-A- 5 138 245
- US-A1- 2006 092 674
- US-A1- 2006 161 180
- US-A1- 2007 244 471
- US-A1- 2007 270 784
- US-A1- 2007 270 790
- US-B2- 6 491 701
- US-B2- 6 491 701

## Description

### RELATED APPLICATIONS

This application claims benefit to U.S. Provisional Patent Application Serial No. 61/079,045, filed on July 8, 2008, and U.S. Provisional Patent Application Serial No. 61/079,416, filed on July 9, 2008, each of which is expressly incorporated herein in its entirety by reference thereto.

### FIELD OF THE INVENTION

The present invention relates to a robotic surgical system comprising a surgical attachment.

### BACKGROUND INFORMATION

Robotic surgical systems may be used to perform various surgical procedures, e.g., laparoscopic procedures. These systems allow, e.g., a surgeon to remotely operate robotic surgical instruments during such procedures. Robotic surgical instruments particularly suited for laparoscopic procedures may include an elongated shaft suitable for insertion into a patient's body, e.g., through a cannula. Such devices may have an end effector at a distal end of the shaft for performing the surgical procedures.

U.S. Patent No. 5,792,135, for example, describes a robotic surgical system having a tubular shaft that rotates and linearly slides with respect to a support bracket on which servomotors are mounted. The system uses a system of cables and pulleys to transfer mechanical power from the servomotors to end effectors mounted at the distal end of a tubular shaft, as well as to actuate the rotation and axial sliding of the shaft.

U.S. Patent No. 6,331,181, for example, describes a robotic surgical system having a plurality of surgical tools mountable onto an instrument holder of a manipulator. A surgical tool includes a probe having an end effector at a distal end and an interface at a proximal end. The manipulator is arranged to orient and translate the mounted surgical tool, e.g., to provide for insertion of a distal portion of the probe, including the end effector, into a patient's body. To actuate the surgical tool, mechanical power is transmitted from servomotors of the manipulator to the removably mounted surgical tool via mechanical coupling of the manipulator to the interface at the proximal end of the probe. The mechanical power is then transferred from the interface to the end effector via a system of cables and pulleys similar to that of U.S. Patent No. 5,792,135.

The robotic surgical systems described above provide actuators that are mounted separately from the insertable shafts. According to these systems, the shafts and end effectors move in relation to the position of the actuators, e.g., during extension of the shaft into the patient's body. This may complicate and/or limit the precise control of the end effectors by requiring complex power transmission systems, e.g., cable and pulley systems. Moreover, servomotor/cable/pulley systems may not provide sufficient power for certain surgical procedures such as, e.g., full thickness resection, anastomosis, and/or transection of thick tissue. This may be due largely to the provision that movement of the surgical robots is controlled by servos, cables, and pulleys rather than other mechanisms capable of potentially providing greater mechanical advantage, and due to motors being located far remotely relative to the end effectors.

Further, generating sufficient mechanical force at the surgical tool or surgical attachment may present difficulty because an electrical power supply appropriate to drive sufficiently powerful electric motors may increase risk to the patient, e.g., from electrical shock. Thus, it may be desirable to limit the level of voltage and/or current being supplied to the end unit and/or locate the actuator at a location distant from the patient. The latter may result in increased complexity and cost and decreased precision as a result of transferring the mechanical power over an extended distance and the difficulties associated therewith, e.g., material flexure, gearing backlash, etc.

US 2007/270784 A1 discloses a handheld, motorised surgical device containing rechargeable battery cells and an additional power source for delivering short bursts of high power to the drive motor.

### SUMMARY

The invention is set out in the appended set of claims. According to the invention, a robotic surgical system comprises a self-contained surgical attachment comprising a surgical tool; an actuator configured to drive the surgical tool; and a power cell for providing electrical power to the actuator, the power cell being configured to accumulate stored electrical energy during an idle state of the actuator and to discharge at least a portion of the stored energy to power the actuator during a peak load of the actuator and the robotic surgical system further comprises: a manipulation unit to which the surgical attachment is removably mounted, wherein the mounting of the surgical attachment to the manipulation unit allows an electrical current to be transferred from the manipulation unit to the surgical attachment to provide electrical power to the actuator and to charge the power cell; a continuous electrical power source coupled to the manipulation unit to provide said electrical power to charge the power cell, wherein the power cell is configured to output a current greater than the current of the continuous electrical power source during the peak load of the actuator; and a control station configured to receive input from an operator and to exchange control signals with the manipulation unit and the surgical attachment, wherein the manipulation unit is configured to position the surgical attachment in relation to a surgical procedure site by translating and/ or rotating the surgical attachment in response to control signals received from the control station.

The power cell may be configured to not provide any current to the actuator during the idle state of the actuator.

The continuous electrical power source may be configured to provide electrical power to the actuator during the idle state of the actuator.

The continuous current source may be configured to provide, along with the power cell, current to the actuator during the peak load of the actuator.

The actuator may include an electric motor.

The surgical system may include multiple surgical attachments.

The surgical attachments may have interchangeable power cells.

The robotic surgical system may include a dock, the surgical attachment being selectively and releasably coupleable to each of the manipulation unit and the dock.

The dock may be configured to provide electrical power to charge the power cell.

The dock may be configured to allow data transfer between the control station and the surgical attachment when the surgical attachment is coupled to the dock.

The data may include a control signal from the control station to cause the surgical attachment to be actuated.

The control signal may cause the surgical attachment to be actuated to a default position.

The data may include at least one of surgical attachment identification data, driver position data corresponding to a position of a driver of the surgical attachment, charge level data corresponding to the charge level of the power cell, and diagnostic data.

Further features and aspects of example embodiments of the present invention are described in more detail below with reference to the appended Figures.

### BRIEF DESCRIPTION OF THE DRAWINGS

Figure 1 is a schematic illustration of a robotic surgical system including a surgical attachment according to an exemplary embodiment of the present invention.
Figure 2 is a perspective view of the surgical attachment illustrated in Figure 1.
Figure 3 is a perspective view of a portion of the surgical attachment illustrated in Figure 1.
Figure 4 is a perspective view of a portion of a surgical attachment.
Figure 5 is a back view of the surgical attachment illustrated in Figure 4.
Figure 6 is a back view of an attachment portion of a manipulation unit.
Figure 7 is a back view of the surgical attachment illustrated in Figures 4 and 5 attached to the manipulation unit illustrated in Figure 6.
Figure 8 is a schematic illustration of a robotic surgical system including a dock.

### DETAILED DESCRIPTION

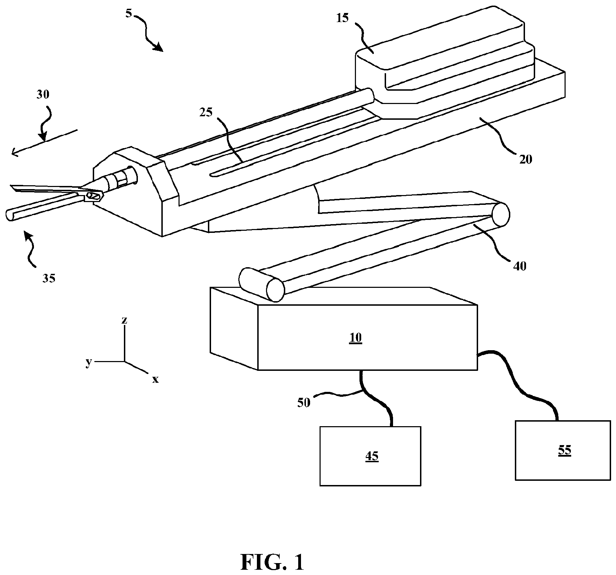

As indicated above, Figure 1 is a schematic illustration of a robotic surgical system 5 according to an exemplary embodiment of the present invention. The surgical system 5 includes a manipulation unit 10 to which a surgical attachment 15 is attached. The manipulation unit 10 is arranged to position the surgical attachment 15 in relation to a surgical procedure site, e.g., a portion of a patient's body, by translating and/or rotating the surgical attachment 15. The surgical attachment 15 is removably attached so that, e.g., the surgical attachment is easily replaced, removed for sterilization between surgical procedures, and/or substituted with a different surgical attachment 15 arranged for a different surgical procedure. The surgical attachment 15 may, however, be permanently attached to the manipulation unit 10. The surgical attachment 15 is mounted onto a slide mount 20 of the manipulation unit 10. The slide mount 20 guides the surgical attachment 15 in the direction of tracks 25 so as to translate the surgical attachment 15 in a distal direction 30. This distal translation allows a distal portion 35 to extend distally away from the manipulator unit 10. This distal extension may be useful, e.g., for insertion of the surgical attachment 15 into a cannula for a laparoscopic surgical procedure. The manipulator unit 10 also positions the surgical attachment 15 with an articulating jointed arm 40. The articulating jointed arm 40 allows precise positioning of the surgical attachment 15 by translating and/or rotating the surgical attachment 15 with respect to all three axes, e.g., x-, y-, and z-axes. The manipulator unit 10 utilizes electric actuators, e.g., electric motors, to position the surgical attachment 15. An electric power source 45, e.g., an electric power grid, is coupled to the manipulator via, e.g., an electrical cord 50 and plug to provide a continuous power supply to the surgical system 5.

The surgical system 5 is operable via a control system or station 55 that communicates with the manipulator unit 10 and/or surgical attachment 15. The control station 55 may send signals via, e.g., wires, fiber optics, wireless transmission, etc., which are received by the manipulator unit 10 and/or surgical attachment 15. The manipulator unit 10 and/or surgical attachment 15 may send signals, e.g., feedback signals, to the control station 55 via, e.g., wires, fiber optics, wireless transmission, etc., which are received by the control station 55. In this regard, the manipulator 10 and/or surgical attachment 15 may be controlled from the control station 50 from substantial distances or in relatively close proximity to the surgical procedure site.

The manipulator unit 10 may be arranged to simultaneously support and/or operate multiple surgical attachments 15, some or all of which may be removable. Multiple surgical attachments 15 may allow for a broader range of procedures to be carried out efficiently as a result of, e.g., not having to switch out surgical attachments 15 to perform different procedures, or minimizing such switching out. The surgical attachments 15 may send identification signals to the control system 55 that identify, e.g., the attachment type, the specific unit serial number, and/or calibration data specific to each surgical attachment 15.

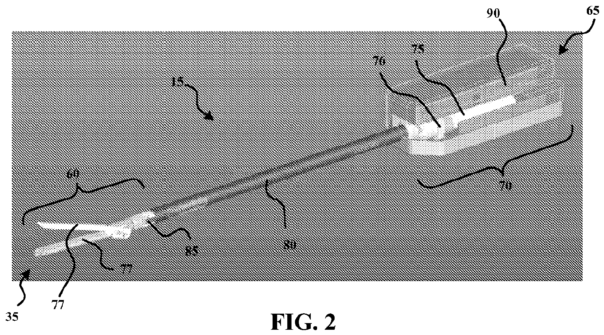

Referring to Figure 2, the surgical attachment 15 includes at the distal end 35 an end effector or surgical tool 60. The surgical tool 60 includes jaws 77 for clamping tissue. It should be appreciated that any surgical tool suitable for robotic surgical procedures may be provided. For example, the surgical attachment 15 may be provided with a cutting and stapling device such as, e.g., described in U.S. Patent No. 7,114,642, which is expressly incorporated herein in its entirety by reference thereto.

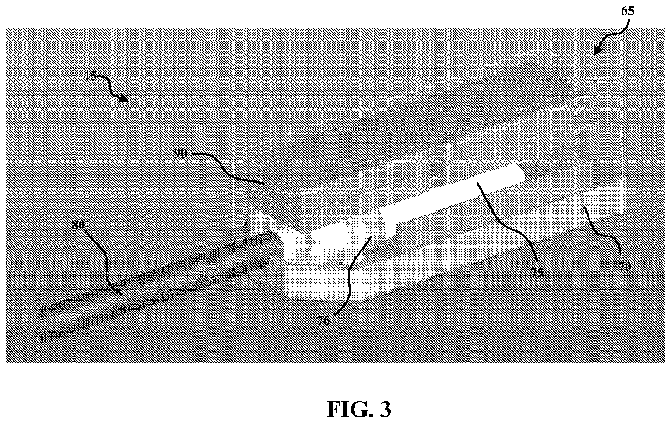

Referring to Figures 2 and 3, the surgical attachment includes at a proximal end 65 a housing 70. The housing 70 includes an actuator 75, e.g., an electric motor, arranged to drive the surgical tool 60. The actuator 75 includes a reduction gear unit 76. It should be appreciated that alternative or additional types of actuators, e.g., linear actuators, may be used. In this regard, mechanical power is transmitted within a shaft 80 that extends between the housing 70 and the surgical tool 60. The mechanical power may be transmitted, e.g., by one or more flexible or rigid power transmission shafts that extend within the shaft 80 and from an output of the actuator 75 to an input of the surgical tool 60. Multiple actuators 75 may be arranged to drive a shared output and/or multiple actuators 75 may be arranged to drive different outputs, depending, e.g., on the nature of the surgical tool 60. For example, a single surgical tool 60 may be arranged as a clamping, cutting, and stapling tool. For this surgical tool 60, a first actuator or actuators 75 may actuate the closing of two jaws 77 to clamp tissue therebetween, and a second actuator or actuators 75 may actuate a cutter and stapler. Further, an actuator or actuators 75 may be used, e.g., to articulate a portion of the surgical tool 60 at a joint 85 and/or to manipulate the shaft 80. For example, the shaft 80 may be rotated about its axis, articulated, and/or bent or flexed if the shaft is so arranged.

Certain procedures, e.g., clamping thick tissue, require a substantial amount of force. To provide such force, the electrically powered actuator or actuators 75 may require a substantial amount of voltage and/or current. To provide electrical power to the actuator or actuators 75 during these procedures, the surgical attachment 15 includes an electrical power cell 90, e.g., a battery, disposed in the housing 70. The electrical power cell 90 may be replaceable or permanent.

When the surgical attachment 15 is attached to the manipulator unit 10, a low voltage/amperage continuous power supply is provided to the surgical attachment 15. The power supply may be transmitted via electrical contacts on the housing 70 that couple with electrical contacts on the slide mount 20 of the manipulation unit 10, or, e.g., a plug connection. The power supply is generated from the electric power source 45. The power may be processed at the manipulator unit 10 by, e.g., conversion from alternating current to direct current and/or regulation of the voltage and/or amperage before being supplied to the surgical attachment 15. As a result of the proximity of the surgical attachment 15 to the surgical procedure site, regulation of the voltage and/or amperage may be suitable to limit the risk of electric shock to the patient or medical personnel in the vicinity of the surgical site. This risk may be further elevated due to, e.g., transmitting high power levels over electrical contacts between the removable surgical attachment 15 and the manipulator unit 10. Thus, if the power transmitted over the contacts is limited, the risk may be reduced.

Although the surgical attachment 15 illustrated in Figure 1 receives power via the manipulator unit 10, it should be appreciated that the surgical attachment 15 may alternatively or additionally receive power separately from the manipulator unit 10 while attached and/or unattached from the manipulator unit 10. For example, the surgical attachment 15 may be permanently or removably attached to a power cord, which may be plugged into, e.g., an alternating current wall outlet. The power cord and/or the surgical attachment 15 may have an arrangement to process the electric power, e.g., conversion from alternating current to direct current and/or regulation of the voltage and/or amperage.

The power supply, although having sufficiently low voltage and/or amperage levels to maintain safety, is sufficient for idling of the actuator or actuators 75, e.g., electric motors, and for other low power applications, e.g., powering sensors and receiving and transmitting control and feedback signals. The power supply also is sufficient to supply the power cell with a continuous low voltage/amperage power supply. This power serves to charge the power cell 90 when the power cell is not being discharged, e.g., during idling of the actuator or actuators 75. Upon sufficient charging, the power cell 90 may discharge at least a portion of the accumulated stored electrical energy during peak operation of the actuator or actuators 75, e.g., when the actuator or actuators 75 drive the surgical tool 60 to clamp a thick section of tissue. After this peak electrical load, e.g., when the actuator or actuators return to an idle state, the power cell 90 ceases discharging and is then recharged by the continuous power supply. After sufficient charging, the power cell 90 may again be used to discharge in order to power the actuator or actuators 75. In this manner, the power cell 90 may be used intermittently to supply power to the actuator or actuators 75 that exceeds the amperage and/or voltage of the continuous power supply from the manipulation unit 10. Although the power cell 90 supplements the continuous power source to power the actuator or actuators 75 during the peak load periods, it should be appreciated that the power cell 90 may be arranged to be the sole source of power for the actuator or actuators 75 during the peak load.

If the actuator or actuators 75 were to consume three watts of power during a peak load that lasts for five seconds and 1.5 watts of power during idling, it may take approximately 100 times the time of the peak load, i.e., 300 seconds, to recharge the power cell 90.

As the power cell 90 allows the surgical attachment 15 to require only a low amperage and/or low voltage power supply from the manipulator unit 10, the actuator or actuators 75 are able to be mounted in the surgical attachment 15 in relatively close proximity to the surgical site with limited risk of electrical shock to the patient and/or medical personnel. This may be a particularly well-suited arrangement for efficient transmission of substantial mechanical force to the surgical tool 60, as the mechanical power may be more directly transmitted as compared to more complex and/or spaced-apart systems, e.g., the servo/cable/pulley systems described above.

Although the surgical attachment 15 illustrated in Figure 1 is mechanically actuated by actuators 75 of the surgical attachment 15, it should be appreciated that the surgical attachment 15 may receive mechanical power from the manipulator unit via, e.g., a gear or driver interface with components that mate when the surgical attachment 15 is attached to the manipulator unit 10. In this regard, the mechanical power transferred via the interface between the surgical attachment 15 and the manipulator unit 10 may be used to drive a process separate from a process driven by the actuators 75 of the surgical attachment 15. Alternatively or additionally, the mechanical power transferred via the interface may supplement the actuators 75 and/or the actuators 75 may supplement the mechanical power transferred via the interface, to drive a given process or processes. For example, the manipulator unit 10 may actuate a clamping process via a mechanical driver or gear interface between the manipulator 10 and the surgical attachment 15, where the actuators 75 selectively provide additional mechanical power based on, e.g., the dynamic mechanical load exceeding a predetermined threshold.

It should be appreciated that multiple power cells 90 may be provided. Moreover, multiple power cells 90 may power a single actuator 75 and/or multiple actuators 75. One or more power cells 90 may charge while one or more other power cells 90 discharge. For example, first power cells 90 may discharge to power a first actuator 75 to drive a first function of the surgical tool 60 at the same time that second power cells 90 are charged for later powering a second actuator 75 to drive a second function of the surgical tool 60. Further, an array of power cells 90 may be arranged such that the number and particular combination of power cells 90 to discharge are selected based on a particular load or procedure. For example, the surgical attachment 15 may have, e.g., six power cells 90 where, for a given procedure, three power cells 90 are assigned to power a first actuator 75, two are assigned to power a second actuator 75, while the sixth power cell 90 charges. One or more power cells 90 may be permanently assigned to one or more actuators 90 and/or some or all of the power cells 90 may be dynamically assigned by, e.g., a control system. Further, the surgical system 5 may be designed to dynamically allocate a power cell 90 or power cells 90 to an actuator 75 based on a sensed load on the actuator 75. Such allocation may be performed by, e.g., a computer and/or circuitry within the surgical attachment 15. A very flexible power system may thus be provided.

The level of charge in the power cell or cells 90 may be monitored by the control system including, e.g., a computer, to determine when the power cell or cells 90 is/are sufficiently charged for a given procedure.

Power cells 90 may be arranged so as to be removable and interchangeable between the same or differently arranged surgical attachments 15. This may allow, e.g., an efficient arrangement where power cells 90 may be installed in more frequently used surgical attachments 15 installed in less frequently used surgical attachments 15 only when needed, thus reducing the total number of power cells 90 required at a given time. A given surgical attachment 15 may be arranged to operate with a variable number of power cells 90 installed, e.g., in slots. For example, a surgical attachment 15 may have three slots to accommodate three power cells 90 that may be required for higher-load procedures, e.g., cutting, clamping, and/or stapling thick tissue, but may operate with only two power cells 90 installed for lower-load procedures, e.g., clamping, cutting, and/or stapling of thin tissue. This may allow for efficient allocation of power cells 90.

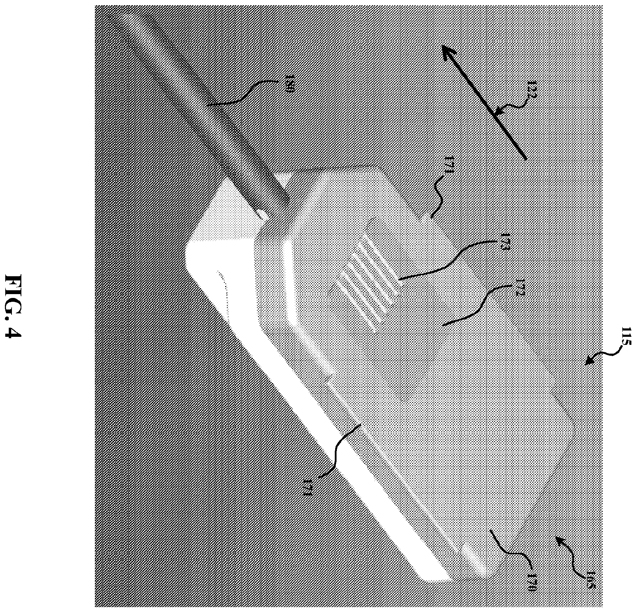

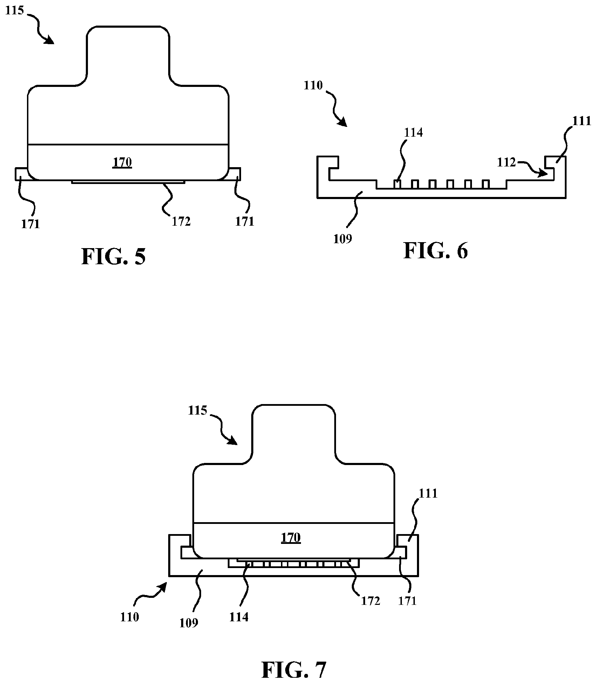

Figures 4 and 5 illustrate a surgical attachment 115. The surgical attachment 115 includes all of the features described above with respect to the surgical attachment 15 and/or features analogous thereto. For example, the surgical attachment 115 includes a housing 170 at a distal end 165, and a shaft 180. In addition, the surgical attachment 115 includes a pair of mounting protrusions or flanges 171 that extend along the sides of the housing 170. Although the mounting protrusions 171 are integrally formed with the housing 170, it should be appreciated that one or both of the mounting protrusions 171 may be formed separately from the housing 170. Moreover, any number of mounting protrusions 171, including a single protrusion 171, may be provided.

The surgical attachment 115 also includes a contact portion 172 that includes a plurality of first electrical contacts 173, in the form of electrically conductive strips. The structure of the contact portion 172 is raised around all but one side of the first electrical contacts 173 to protect the first electrical contacts 173 from damage and/or abrasion from, e.g., handling and/or storing the surgical attachment 115. The electrical contacts 173 are electrically coupled to the internal components of the surgical attachment 115 contained in the housing 172. The electrical contacts 173 supply electrical energy to the power cell 90 and to motors and actuators provided in the surgical attachment 115, as well as provide for communication between the surgical attachment 115 and a remote control system.

Figure 6 is a back view of an attachment portion 109 of a manipulation unit 110. The manipulation unit 110 includes the same or analogous features as those described above with respect to the manipulation unit 10. The attachment portion 109 may, e.g., be part of a carriage that carries the surgical attachment 115 along a slide mount 20 such as that shown in Figure 1. The attachment portion 109 also includes a pair of flanged attachment arms 111 that define channels 112. The attachment portion 109 also includes a plurality of second electrical contacts 114.

Figure 7 is a back view of the surgical attachment 115 illustrated in Figures 4 and 5 attached to the attachment portion 109 of the manipulation unit 110 illustrated in Figure 6. The surgical attachment 115 is inserted into the attachment portion 109 by engaging the mounting protrusions 171 into respective channels 112 formed by the flanged attachment arms 111 and then sliding the surgical attachment 115 in an insertion direction 122 (shown in Figure 4). Although the insertion direction 122 is distally directed and parallel to the axis of the shaft 180, it should be appreciated that other insertion directions may be provided. For example, transversely extending mounting protrusions 171 may be provided that mate with transversely extending channels 112 such that the insertion direction 122 is transverse to the shaft 180. The attachment portion may be secured in the installed position by any appropriate mechanism, e.g., a latch.

During insertion of the surgical attachment 115 into the attachment portion 109, the second electrical contacts 114 engage and slide along the first electrical contacts 173. In this regard, the second electrical contacts 114 are spring-loaded, e.g., by taking the form of electrically conductive spring arms, toward the first electrical contacts 114 so as to maintain reliable contact with the first electrical contacts 114. The coupling of the first electrical contacts 173 and the second electrical contacts 114 when the surgical attachment 115 is attached allows, e.g., signals and/or electrical current to flow over separate channels, each of which may be defined by an individual pairing of a first electrical contact 173 and second electrical contact 114. In this manner, e.g., electrical power and/or control signals/information may be provided/exchanged between the surgical attachment 115 and the manipulation unit 110.

It should be appreciated, however, that the manipulation unit 10, 110 and the surgical attachment 15, 115 may communicate by additional or alternative mechanisms, e.g., fiber optics and/or wireless communication devices. Electrical power transmission may be provided by additional or alternative mechanisms, e.g., inductive coupling between the surgical attachment 15, 115 and the manipulation unit 10, 110. In this regard, hard electrical connections between the surgical attachment 15, 115 and the manipulation unit 10, 110 may be dispensed with entirely, e.g., where communication of control/feedback signals is transmitted wirelessly and/or optically and electricity is provided inductively to power the surgical attachment 15, 115, including, e.g., inductively charging the power cell or cells 90.

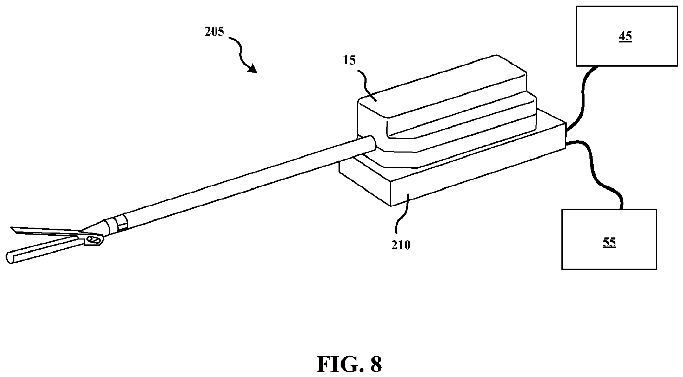

Further, the surgical device may be charged and/or controlled when not mounted for performing a surgical procedure. For example, referring to the docking system 205 schematically illustrated in Figure 8, the surgical attachment 15 may be mounted in a dock 210, e.g., a charging dock, separate from or attached to the manipulator unit 10. Such a dock 210 may couple with the surgical attachment 15 via the same and/or analogous mechanisms as described above with respect to the coupling and/or communication between the surgical attachment 15 and the manipulator unit 10. In this manner, the surgical attachment 15 may, e.g., be charged, be actuated, and/or exchange signals with a control system 55, e.g., a computer, when the surgical attachment 15 is not mounted for performing a procedure. For example, the surgical attachment 15 may be detached from the slide mount 20 after a procedure and placed into the dock 210. While in the dock 210, the surgical attachment 15 may, e.g., receive electrical power to recharge the power cell or cells 90, e.g., from power source 45. Further, the dock 210 may be coupled to a control system, e.g., control station 55, such that the surgical attachment 15 may be actuated, e.g., to a default position, and/or receive data including, e.g., device identification data, driver position data, charge level data for the power cell or cells 90, and/or diagnostic data.

Although the dock 210 is schematically illustrated as being connected to the same power source 45 and control system or station 55 as the manipulator unit 10, it should be appreciated that the dock 210 may receive power from a different power source and/or may communicate with a different control system or station as the manipulator unit.

As indicated above, many current surgical robots may not be particularly suited to perform procedures that require a large amount of force. The power cell or cells 90 may be self-contained within the surgical attachment 15. This would provide for a more self-contained surgical attachment 15 that would provide electrical power to the actuators 75 during peak output, independently of and in addition to the continuous power from the manipulator unit 10 to power high-powered surgical instruments. Further, due to the layer of the surgical attachment and the serial communications with the manipulator unit and/or control station 55, additional degrees of movement and/or additional steps to carry out a procedure may be provided for.

Although the present invention has been described with reference to particular examples and exemplary embodiments, it should be understood that the foregoing description is in no manner limiting. Moreover, the features described herein may be used in any combination.

## Claims

1. A robotic surgical system (5) comprising:
a self-contained surgical attachment (15,115) comprising:
a surgical tool (60);
an actuator (75) configured to drive the surgical tool; and
a power cell (90) for providing electrical power to the actuator, the power cell being configured to accumulate stored electrical energy during an idle state of the actuator and to discharge at least a portion of the stored energy to power the actuator during a peak load of the actuator,
**characterized in that** the robotic surgical system further comprises:
a manipulation unit (10,110) to which the surgical attachment is removably mounted, wherein the mounting of the surgical attachment to the manipulation unit allows an electrical current to be transferred from the manipulation unit to the surgical attachment to provide electrical power to the actuator and to charge the power cell;
a continuous electrical power source (45) coupled to the manipulation unit (10) to provide said electrical power to charge the power cell (90), wherein the power cell (90) is configured to output a current greater than the current of the continuous electrical power source during the peak load of the actuator (75); and
a control station (55) configured to receive input from an operator and to exchange control signals with the manipulation unit and the surgical attachment,
wherein the manipulation unit (10,110) is configured to position the surgical attachment in relation to a surgical procedure site by translating and/or rotating the surgical attachment in response to control signals received from the control station (55).

2. The robotic surgical system according to claim 1, wherein the power cell (90) is configured to not provide any current to the actuator (75) during the idle state of the actuator.

3. The robotic surgical system according to claim 1, wherein the continuous electrical power source (45) is configured to provide electrical power to the actuator (75) during the idle state of the actuator.

4. The robotic surgical system according to claim 1, wherein the continuous current source (45) is configured to provide, along with the power cell (90), current to the actuator (75) during the peak load of the actuator.

5. The robotic surgical system according to any preceding claim, wherein the actuator (75) includes an electric motor.

6. The robotic surgical system according to any preceding claim, wherein the robotic surgical system includes multiple surgical attachments.

7. The robotic surgical system according to claim 6, wherein the surgical attachments include interchangeable power cells (90).

8. The robotic surgical system according to any preceding claim, further comprising a dock (210), the surgical attachment being selectively and releasably coupleable to each of the manipulation unit and the dock.

9. The robotic surgical system according to claim 8, wherein the dock (210) is configured to provide electrical power to charge the power cell (90).

10. The robotic surgical system according to claim 8, wherein the dock (210) is configured to allow data transfer between the control station (55) and the surgical attachment when the surgical attachment is coupled to the dock.

11. The robotic surgical system according to claim 10, wherein the data includes a control signal from the control station to cause the surgical attachment to be actuated.

12. The robotic surgical system according to claim 11, wherein the control signal causes the surgical attachment to be actuated to a default position.

13. The robotic surgical system according to claim 10, wherein the data includes at least one of surgical attachment identification data, driver position data corresponding to a position of a driver of the surgical attachment, charge level data corresponding to the charge level of the power cell, and diagnostic data.

## Patentansprüche

1. Robotergestütztes chirurgisches System (5) umfassend:
eine eigenständige chirurgische Befestigung (15, 115) umfassend:
ein chirurgisches Instrument (60);
ein Stellglied (75), das konfiguriert ist, um das chirurgische Instrument anzutreiben; und
eine Energiezelle (90) zum Bereitstellen von elektrischer Energie an das Stellglied, wobei die Energiezelle konfiguriert ist, um gespeicherte elektrische Energie während eines Ruhezustands des Stellglieds zu speichern und mindestens einen Teil der gespeicherten Energie zu entladen, um das Stellglied während einer Spitzenlast des Stellglieds mit Strom zu versorgen,
**dadurch gekennzeichnet, dass** das robotergestützte chirurgische System weiter umfasst:
eine Manipulationseinheit (10, 110), an der die chirurgische Befestigung entfernbar befestigt ist, wobei die Befestigung der chirurgischen Befestigung an der Manipulationseinheit es ermöglicht, einen elektrischen Strom von der Manipulationseinheit auf die chirurgische Befestigung zu übertragen, um elektrische Energie an das Stellglied bereitzustellen und die Energiezelle aufzuladen;
eine kontinuierliche elektrische Energiequelle (45), die mit der Manipulationseinheit (10) gekoppelt ist, um die elektrische Energie bereitzustellen, um die Energiezelle (90) aufzuladen, wobei die Energiezelle (90) konfiguriert ist, um während der Spitzenlast des Stellglieds (75) einen Strom auszugeben, der größer als der Strom der kontinuierlichen elektrischen Energiequelle ist; und
eine Steuerstation (55), die konfiguriert ist, um eine Eingabe von einem Bediener zu empfangen und Steuersignale mit der Manipulationseinheit und der chirurgischen Befestigung auszutauschen,
wobei die Manipulationseinheit (10, 110) konfiguriert ist, um die chirurgische Befestigung in Bezug auf eine chirurgische Eingriffsstelle durch Verschieben und/oder Drehen der chirurgischen Befestigung als Reaktion auf von der Steuerstation (55) empfangenen Steuersignalen zu positionieren.

2. Robotergestütztes chirurgisches System nach Anspruch 1, wobei die Energiezelle (90) konfiguriert ist, um an das Stellglied (75) während des Ruhezustands des Stellglieds keinen Strom bereitzustellen.

3. Robotergestütztes chirurgisches System nach Anspruch 1, wobei die kontinuierliche elektrische Energiequelle (45) konfiguriert ist, um an das Stellglied (75) während des Ruhezustands des Stellglieds elektrische Energie bereitzustellen.

4. Robotergestütztes chirurgisches System nach Anspruch 1, wobei die kontinuierliche Stromquelle (45) konfiguriert ist, um zusammen mit der Energiezelle (90) an das Stellglied (75) während der Spitzenlast des Stellglieds Strom bereitzustellen.

5. Robotergestütztes chirurgisches System nach einem vorstehenden Anspruch, wobei das Stellglied (75) einen Elektromotor aufweist.

6. Robotergestütztes chirurgisches System nach einem vorstehenden Anspruch, wobei das robotergestützte chirurgische System mehrere chirurgische Befestigungen aufweist.

7. Robotergestütztes chirurgisches System nach Anspruch 6, wobei die chirurgischen Befestigungen austauschbare Energiezellen (90) aufweisen.

8. Robotergestütztes chirurgisches System nach einem vorstehenden Anspruch, weiter umfassend ein Dock (210), wobei die chirurgische Befestigung selektiv und lösbar mit jedem von der Manipulationseinheit und dem Dock koppelbar ist.

9. Robotergestütztes chirurgisches System nach Anspruch 8, wobei das Dock (210) konfiguriert ist, um elektrische Energie bereitzustellen, um die Energiezelle (90) aufzuladen.

10. Robotergestütztes chirurgisches System nach Anspruch 8, wobei das Dock (210) konfiguriert ist, um Datenübertragung zwischen der Steuerstation (55) und der chirurgischen Befestigung zu ermöglichen, wenn die chirurgische Befestigung mit dem Dock gekoppelt ist.

11. Robotergestütztes chirurgisches System nach Anspruch 10, wobei die Daten ein Steuersignal von der Steuerstation aufweisen, um zu bewirken, dass die chirurgische Befestigung betätigt wird.

12. Robotergestütztes chirurgisches System nach Anspruch 11, wobei das Steuersignal bewirkt, dass die chirurgische Befestigung in eine Standardposition betätigt wird.

13. Robotergestütztes chirurgisches System nach Anspruch 10, wobei die Daten mindestens eines von Identifikationsdaten der chirurgischen Befestigung, Antriebspositionsdaten, die einer Position eines Antriebs der chirurgischen Befestigung entsprechen, Ladezustandsdaten, die dem Ladezustand der Energiezelle entsprechen, und Diagnosedaten aufweisen.

## Revendications

1. Système chirurgical robotique (5) comprenant :
une fixation chirurgicale auto-contenue (15, 115) comprenant :
un outil chirurgical (60) ;
un actionneur (75) configuré pour entraîner l'outil chirurgical ; et
une cellule d'alimentation (90) pour fournir une alimentation électrique à l'actionneur, la cellule d'alimentation étant configurée pour accumuler de l'énergie électrique stockée pendant un état au repos de l'actionneur et pour décharger au moins une partie de l'énergie stockée pour alimenter l'actionneur pendant une charge de pointe de l'actionneur,
**caractérisé en ce que** le système chirurgical robotique comprend en outre :
une unité de manipulation (10, 110) sur laquelle la fixation chirurgicale est montée de manière amovible, dans lequel le montage de la fixation chirurgicale sur l'unité de manipulation permet à un courant électrique d'être transféré de l'unité de manipulation à la fixation chirurgicale afin de fournir une alimentation électrique à l'actionneur et de charger la cellule d'alimentation ;
une source d'alimentation électrique continue (45) couplée à l'unité de manipulation (10) pour fournir ladite alimentation électrique pour charger la cellule d'alimentation (90), dans lequel la cellule d'alimentation (90) est configurée pour délivrer en sortie un courant supérieur au courant de la source d'alimentation électrique continue pendant la charge de pointe de l'actionneur (75) ; et
un poste de commande (55) configuré pour recevoir une entrée d'un opérateur et pour échanger des signaux de commande avec l'unité de manipulation et la fixation chirurgicale,
dans lequel l'unité de manipulation (10, 110) est configurée pour positionner la fixation chirurgicale par rapport à un site d'intervention chirurgicale en déplaçant en translation et/ou en faisant tourner la fixation chirurgicale en réponse à des signaux de commande reçus du poste de commande (55).

2. Système chirurgical robotique selon la revendication 1, dans lequel la cellule d'alimentation (90) est configurée pour ne fournir aucun courant à l'actionneur (75) pendant l'état au repos de l'actionneur.

3. Système chirurgical robotique selon la revendication 1, dans lequel la source d'alimentation électrique continue (45) est configurée pour fournir une alimentation électrique à l'actionneur (75) pendant l'état au repos de l'actionneur.

4. Système chirurgical robotique selon la revendication 1, dans lequel la source d'alimentation continue (45) est configurée pour fournir, avec la cellule d'alimentation (90), un courant à l'actionneur (75) pendant la charge de pointe de l'actionneur.

5. Système chirurgical robotique selon l'une quelconque des revendications précédentes, dans lequel l'actionneur (75) inclut un moteur électrique.

6. Système chirurgical robotique selon l'une quelconque des revendications précédentes, dans lequel le système chirurgical robotique inclut plusieurs fixations chirurgicales.

7. Système chirurgical robotique selon la revendication 6, dans lequel les fixations chirurgicales incluent des cellules d'alimentation interchangeables (90).

8. Système chirurgical robotique selon l'une quelconque des revendications précédentes, comprenant en outre une station d'accueil (210), la fixation chirurgicale pouvant être couplée de manière sélective et libérable à chacune de l'unité de manipulation et de la station d'accueil.

9. Système chirurgical robotique selon la revendication 8, dans lequel la station d'accueil (210) est configurée pour fournir une alimentation électrique pour charger la cellule d'alimentation (90).

10. Système chirurgical robotique selon la revendication 8, dans lequel la station d'accueil (210) est configurée pour permettre un transfert de données entre le poste de commande (55) et la fixation chirurgicale lorsque la fixation chirurgicale est couplée à la station d'accueil.

11. Système chirurgical robotique selon la revendication 10, dans lequel les données incluent un signal de commande provenant du poste de commande pour provoquer l'actionnement de la fixation chirurgicale.

12. Système chirurgical robotique selon la revendication 11, dans lequel le signal de commande provoque l'actionnement de la fixation chirurgicale dans une position par défaut.

13. Système chirurgical robotique selon la revendication 10, dans lequel les données incluent au moins une de données d'identification de fixation chirurgicale, de données de position de pilote correspondant à une position d'un pilote de la fixation chirurgicale, de données de niveau de charge correspondant au niveau de charge de la cellule d'alimentation, et de données de diagnostic.
